# EUROPÄISCHE PATENTSCHRIFT

(11) **EP 1 391 186 B1**
(45) Veröffentlichungstag und Bekanntmachung des Hinweises auf die Patenterteilung: **14.02.2007**
(21) Anmeldenummer: 03016039.4
(22) Anmeldetag: 15.07.2003
(51) Int. Cl.: A61F 2/28, B21D 41/02, B21D 41/04, A61B 17/80

(54) **Verfahren zum Herstellen eines rohrförmigen Platzhalters, und Platzhalter**
Method for manufacturing a tubular spacer, and spacer
Procédé de fabrication d'un écarteur tubulaire, et écarteur

(30) Priorität: 21.08.2002 DE 10238306
(43) Veröffentlichungstag der Anmeldung: 25.02.2004
(73) Patentinhaber: BIEDERMANN MOTECH GmbH, 78054 VS-Schwenningen (DE)
(72) Erfinder: Biedermann, Lutz, 78048 VS-Villingen (DE); Harms, Jürgen, 76227 Karlsruhe (DE); Rapp, Helmar, 78652 Deisslingen (DE)
(74) Vertreter: Hofer, Dorothea

(56) Entgegenhaltungen:
- EP-A- 0 268 115
- EP-A- 0 853 931
- WO-A-00/24341
- WO-A-02/34168
- WO-A-99/32055
- WO-A-99/51171
- WO-A-02/085552
- WO-A-03/039784
- DE-A- 2 842 847
- DE-C- 19 504 867
- DE-U- 29 501 042
- FR-A- 1 527 214
- US-A- 3 710 789
- US-A- 5 211 664
- US-A- 5 380 328
- US-A1- 2001 035 038

## Beschreibung

Die Erfindung betrifft ein Verfahren zum Herstellen eines rohrförmigen, Ausnehmungen in seiner Wandung aufweisenden Platzhalters für Knochendefekte, der an seinem einen ersten Ende einen ersten Rand und an seinem gegenüberliegenden zweiten Ende einen zweiten Rand aufweist.

Die Erfindung betrifft ferner einen Platzhalter mit einem rohrförmigen, Ausnehmungen aufweisenden Mantel mit einem ersten Rand an seiner ersten Stirnfläche und einem zweiten Rand an seiner gegenüberliegenden zweiten Stirnfläche.

Aus der DE 195 04 867 C ist ein solcher Platzhalter bekannt. Dieser dient insbesondere zum Ersetzen eines Wirbels oder einer Bandscheibe. Der Platzhalter ist aus einem zylindrischen Rohr gebildet, welches an seinen beiden gegenüberliegenden Stirnflächen den gleichen Durchmesser aufweist. Aus der EP 0 720 840 A ist es bekannt, zum Verbinden von Wirbeln mit unterschiedlicher Größe und unterschiedlichem Querschnitt einen Platzhalter auszubilden, der wenigstens zwei mantelförmige Elemente mit unterschiedlichem Querschnitt und ein Verbindungsstück zum Verbinden der Elemente aufweist. Die einzelnen Elemente sind jeweils wieder zylinderförmig ausgebildet. Die bekannten Platzhalter sind hervorragend für die Wirbelsäulenchirurgie, jedoch nur bedingt als Platzhalter bei Röhrenknochen einsetzbar.

Aufgabe der Erfindung ist es, ein Verfahren der eingangs beschriebenen Art zu schaffen, mit dem sich ein Platzhalter herstellen läßt, der besonders im Bereich der Röhrenknochenchirurgie Anwendung finden kann. Ferner soll ein Platzhalter der eingangs beschriebenen Art geschaffen werden.

Diese Aufgabe wird durch ein Verfahren nach Patentanspruch 1 bzw. 2 bzw. einen Platzhalter nach Patentanspruch 7 bzw. 8 gelöst.

Weiterbildungen des Verfahrens und der Vorrichtung sind in den Unteransprüchen gekennzeichnet.

Mit dem Verfahren lassen sich Platzhalter herstellen, die an den beiden gegenüberliegenden Enden an die jeweiligen zu verbindenden Knochenteile angepaßte Durchmesser aufweisen.

Weitere Merkmale und Zweckmäßigkeiten der Erfindung ergeben sich aus der Beschreibung von Ausführungsbeispielen anhand der Figuren. Von den Figuren zeigen:
- Fig. 1: eine Seitenansicht eines Röhrenknochens mit eingesetztem Platzhalter;
- Fig. 2: eine vergrößerte Darstellung, wobei der Knochen geschnitten gezeigt ist;
- Fig. 3: eine schematische Darstellung einer Vorrichtung zum Ausführen des Verfahrens;
- Fig. 4: eine schematische Darstellung einer weiteren Vorrichtung zur Durchführung des Verfahrens;
- Fig. 5a) bis 5d): Querschnitte der Endflächen der hergestellten Platzhalter;
- Fig. 6a) bis 6d): einen zylindrischen Platzhalter und drei ver schiedene mit dem Verfahren hergestellte erfindungsgemäße Platzhalter;
- Fig. 7: eine abgewandelte Form eines Platzhalters; und
- Fig. 8: eine weitere abgewandelte Form des Platzhalters.

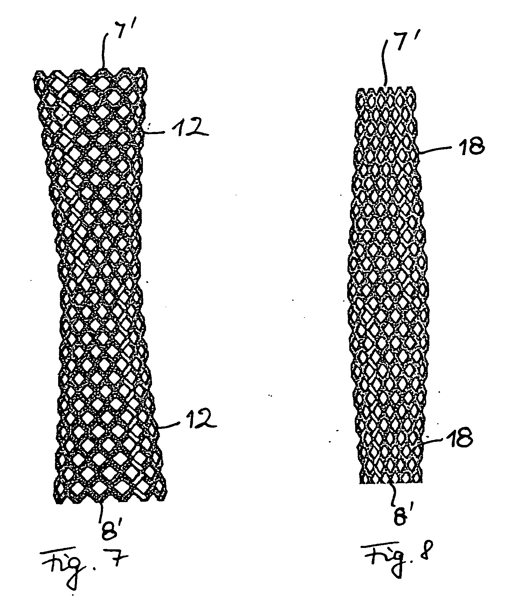

Als Ausgangsmaterial dient, wie am besten auf Fig. 6 ersichtlich ist, ein zylinderförmiges Rohr mit einer Länge, die gleich dem Abstand der miteinander zu verbindenden Knochenteile bzw. der Länge des zu überbrückenden Stückes entspricht.

Der Zylinder weist Ausnehmungen auf, um ein Einwachsen von Knochenmaterial zu erleichtern. In den Figuren ist eine besonders bevorzugte Ausführungsform des Ausgangsmaterials gezeigt. Der zunächst zylindrisch ausgebildete Mantel 1 weist sich mit ihrer Längsdiagonalen parallel zur Mantelachse 2 erstreckende rautenförmige Ausnehmungen 3, 4 auf. Jeweils benachbarte Reihen solcher Rauten sind in Richtung der Mantelachse 2 um eine halbe Rautenhöhe versetzt. Dadurch wird ein Netz von sich unter einem spitzen Winkel schneidenden Bandstreifen 5, 6 gebildet, die unter jeweils gleichgroßen Winkeln gegen die Längsdiagonale der Rauten 3, 4 geneigt sind. Der obere Rand 7 und der untere Rand 8 erstrecken sich jeweils in einer Ebene senkrecht zu der Längsachse 2.

In einer ersten Ausführungsform eines herzustellenden Platzhalters, der in Fig. 6a) dargestellt ist, soll der untere Rand Form und Größe des ursprünglichen unteren Randes des Ausgangszylinders beibehalten, während der obere Rand 7' einen um ein vorbestimmtes Maß größeren Durchmesser aufweisen soll.

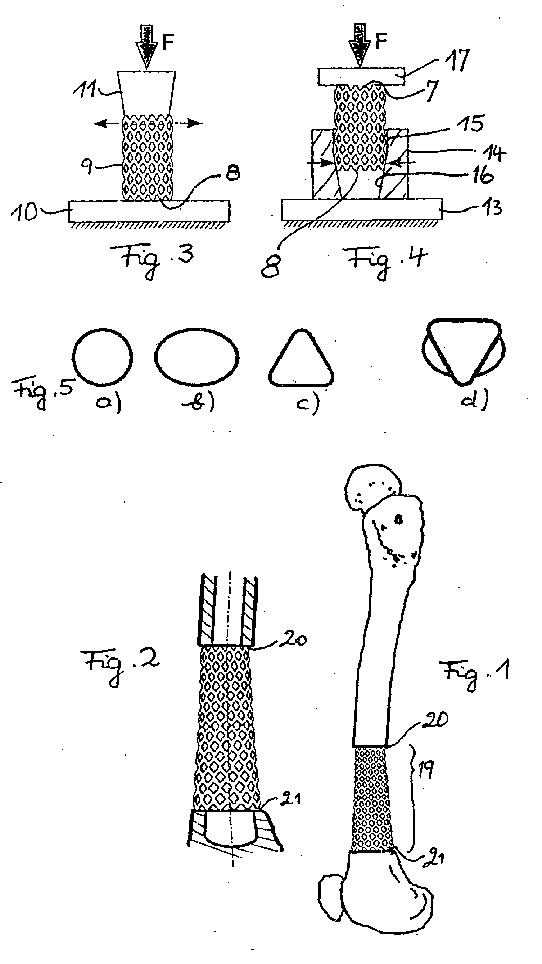

Zu diesem Zweck wird der das Ausgangsmaterial bildende Zylinder 9 mit seinem unteren Rand auf ein Widerlager 10 aufgesetzt. Wie am besten aus Fig. 3 ersichtlich ist, wird in die von dem oberen Rand 7 begrenzte Stirnfläche ein Dorn 11 eingesetzt und mittels einer schematisch angedeuteten Kraft F, die in Richtung der Mantelachse 2 in Richtung des Widerlagers 10 wirkt, so weit in Richtung zum unteren Rand 8 eingetrieben, bis die in Fig. 6a) dargestellte gewünschte Aufweitung erreicht ist. Wie in Fig. 6a) gezeigt, erfolgt durch die Verwendung des Dorns zur Aufweitung die Aufweitung derart, daß sich der Abschnitt 12, in den der Dorn eingetrieben ist, der Form des Dornes anpaßt. Wie in Fig. 3 gezeigt, weist der Dorn eine kegelstumpfförmige Form auf, so daß der Abschnitt 12 entsprechend ausgebildet wird und der obere Rand einen gegenüber dem unteren Rand 8 entsprechend vergrößerten Durchmesser aufweist.

In einer anderen in Fig. 6b) gezeigten Ausführungsform ist es gewünscht, den an den oberen Rand 7 angrenzenden Abschnitt 12' unverändert zu lassen, so daß also insbesondere der Durchmesser des oberen Randes unverändert bleibt. Der untere Rand 8 des Ausgangszylinders 9 soll hingegen verengt werden.

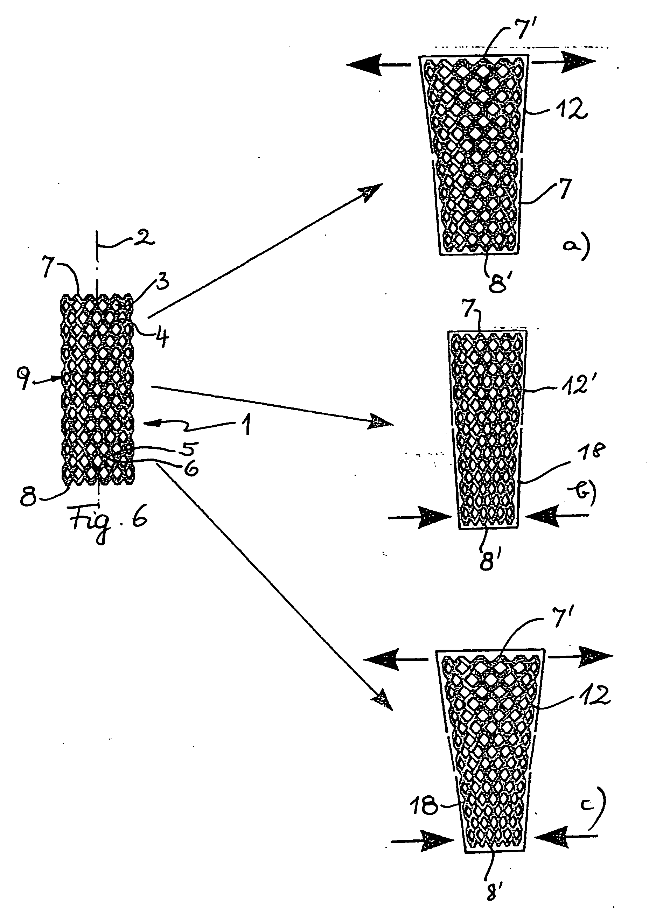

Zu diesem Zweck wird ein Widerlager 13 verwendet, welches einen Hohlstempel 14 aufweist. Dieser besitzt an seinem dem eigentlichen Widerlager 13 gegenüberliegenden freien Enden einen Abschnitt 15, dessen Durchmesser und Form den äußeren Abmessungen des Ausgangszylinders 9 entsprechen. Daran an schließt sich ein zweiter Abschnitt 16, der zum Grund des eigentlichen Widerlagers 13 hin kegelstumpfförmig verengt ausgebildet ist. Der Ausgangszylinder 9 wird in der in Fig. 4 gezeigten Weise mit seinem unteren Rand 8 zum eigentlichen Widerlager hin gerichtet eingebracht. An dem gegenüberliegenden oberen Rand 7 greift ein oberes Widerlager 17 an. Über dieses wird eine schematisch angedeutete Kraft F in Richtung zum Widerlager 13 hin aufgebracht, die bewirkt, daß der untere Rand 8 und der daran anschließende Bereich des Zylinders auf ein gewünschtes vorbestimmtes Maß verengt wird, so daß ein in Fig. 6b) gezeigter verengter Abschnitt 18 entsteht. Bei der in Fig. 4 gezeigten Ausführungsform weist das Widerlager 17 einen in den Zylinder hineingerichteten Führungszylinder auf.

In einer in Fig. 6c) gezeigten weiter abgewandelten Ausführungsform wird gewünschtenfalls in einem ersten Schritt der obere Rand 7 des Ausgangszylinders 9 in Übereinstimmung mit dem Ausführungsbeispiel nach Fig. 6a) aufgeweitet zum Erzeugen eines Abschnittes 12. Die Aufweitung erfolgt anhand des in Fig. 3 beschriebenen Verfahrens. Anschließend wird der untere Rand 8 mit der Vorrichtung und dem Verfahren nach Fig. 4 verengt, so daß der untere Rand und der daran angrenzende Bereich die verengte Form des Abschnittes 18 in Fig. 6b) annimmt.

Auf diese Weise ist es möglich, aus einem Ausgangszylinder 9 unterschiedlich geformte Platzhalter zu schaffen, die an den jeweiligen zu überbrückenden Bereich 19 zwischen zwei Knochenenden 20, 21 unterschiedlicher Form und Größe angepaßt sind.

Der Ausgangszylinder 9 kann verschiedene vorgefertigte Querschnittsformen besitzen, wobei bevorzugt ein kreisförmiger Querschnitt gewählt ist. Dorn 11 und Hohlstempel 14 können ebenfalls einen kreisförmigen Querschnitt aufweisen, so daß die erzeugten Abschnitt 12 und 18 die in Fig. 5a) gezeigte kreisförmige Form haben. Alternativ kann der Querschnitt von Dorn 11 und/oder Hohlstempel 14 jede gewünschte andere Form besitzen, insbesondere die ovale oder dreieckige Form, so daß der bearbeitete Abschnitt die in Fig. 5b), 5c) gezeigte ovale oder dreieckige Querschnittsform aufweisen kann. Ist eine unterschiedliche Verformung von dem oberen Rand 7 und dem unteren Rand 8 gewünscht, ist es möglich, Dorn 11 und Hohlstempel 14 auch mit unterschiedlichen Querschnittsformen zu wählen, so daß beispielsweise wie in Fig. 5d) gezeigt, der eine Rand oval und der andere dreiecksförmig ausgebildet ist.

Wie oben ausgeführt, kann der Ausgangszylinder 9 verschiedene Ausbildungen der Ausnehmungen 3, 4 aufweisen. Die Ausbildung als rautenförmige Öffnungen hat den besonderen Vorteil, daß auch die durch das Aufweiten bzw. Verengen verformten Ränder 7', 8' jeweils einen zackenförmigen Rand besitzen, der eine besonders stabile Verbindung zwischen angrenzenden Knochenenden 20, 21 und Rändern 7', 8' zur Folge hat.

In Fig. 7 ist ein Ausführungsbeispiel gezeigt, bei dem sowohl der obere Rand als auch der untere Rand mit dem anhand von Fig. 3 beschriebenen Verfahrensschritt aufgeweitet wurden, so daß der Platzhalter zu beiden Enden hin konisch aufgeweitete Abschnitte 12 besitzt. In Fig. 8 ist ein Ausführungsbeispiel gezeigt, bei dem die einander gegenüberliegenden Enden mit dem anhand von Fig. 4 beschriebenen Verfahren verengt wurden, so daß der Platzhalter in seiner Mitte die Abmessungen des Ausgangszylinders 9 besitzt, und die beiden an die Mitte angrenzenden Abschnitte zu dem jeweiligen Rand 7', 8' hin verengte Abschnitte 18 besitzen.

Die in Fig. 3 und 4 gezeigten Vorrichtungen sind lediglich schematisch gezeigt. Das Widerlager 10 und der Dorn 11 einerseits und das obere Widerlager 17 und Widerlager 13 mit Hohlstempel 14 andererseits können jeweils über Hebel verbunden sein nach Art einer Kniehebelpresse.

Als Materialien für die Platzhalter werden körperverträgliche Materialien gewählt, insbesondere Stahl oder Titan und die entsprechenden Legierungen. Je nach Ausmaß der Aufweitung oder Verengung kann gewünschtenfalls zum Beseitigen der Materialspannung eine Wärmebehandlung durchgeführt werden.

Bei der Verwendung der beschriebenen Platzhalter erfolgt die zusätzliche Absicherung zur Lastübertragung in an sich bekannter Weise. So wird beispielsweise zum Erzeugen einer Zugkraft zwischen den beiden Knochenenden eine Platte angeschraubt, oder die Knochenteile werden in bekannter Weise durch einen Markraumnagel zum Erzeugen einer Kompression verbunden.

## Patentansprüche

1. Verfahren zum Herstellen eines rohrförmigen, Ausnehmungen in seiner Wandung aufweisenden Platzhalters zum Verbinden von Knochenteilen mit seinen gegenüberliegenden Stirnflächen, der an seinem einen ersten Ende einen ersten Rand (7) und an seinem gegenüberliegenden zweiten Ende einen zweiten Rand (8) aufweist, mit den Schritten:
- Bilden eines zylinderförmigen Rohres (9) mit einer Länge, die dem Abstand miteinander zu verbindender Knochenteile entspricht und
- Aufweiten des zylinderförmigen Rohres (9) von wenigstens einem Ende her.

2. Verfahren zum Herstellen eines rohrförmigen, Ausnehmungen in seiner Wandung aufweisenden Platzhalters zum Verbinden von Knochenteilen mit seinen gegenüberliegenden Stirnflächen, der an seinem einen ersten Ende einen ersten Rand (7) und an seinem gegenüberliegenden zweiten Ende einen zweiten Rand (8) aufweist, mit den Schritten:
- Bilden eines zylinderförmigen Rohres (9) mit einer Länge, die dem Abstand miteinander zu verbindender Knochenteile entspricht und
- Verengen des zylinderförmigen Rohres (9) von wenigstens einem Ende her.

3. Verfahren nach einem der Ansprüche 1 oder 2, **dadurch gekennzeichnet, daß** das Aufweiten von einem Ende und das Verengen von dem anderen Ende her erfolgt.

4. Verfahren nach einem der Ansprüche 1 bis 3, **dadurch gekennzeichnet, daß** der Querschnitt des aufgeweiteten und/oder des verengten Teiles von dem nicht verformten Querschnitt abweicht.

5. Verfahren nach einem der Ansprüche 1 bis 4, **dadurch gekennzeichnet, daß** das Aufweiten durch Eindrücken eines Dornes (11) erfolgt.

6. Verfahren nach einem der Ansprüche 2 bis 5, **dadurch gekennzeichnet, daß** das Verengen durch Eindrücken des Endes in eine Hohlform (14) erfolgt.

7. Platzhalter zum Verbinden von Knochenteilen mit seinen gegenüberliegenden Stirnflächen, mit einem rohrförmigen, Ausnehmungen aufweisenden Mantel (1) mit einem ersten Rand (7) an seiner ersten Stirnfläche und einem zweiten Rand (8) an seiner gegenüberliegenden zweiten Stirnfläche, **dadurch gekennzeichnet, daß** der rohrförmige Mantel (1) durch einen bei der Herstellung an wenigstens einem Ende aufgeweiteten Zylinder gebildet ist.

8. Platzhalter zum Verbinden von Knochenteilen mit seinen gegenüberliegenden Stirnflächen, mit einem rohrförmigen, Ausnehmungen aufweisenden Mantel (1) mit einem ersten Rand (7) an seiner ersten Stirnfläche und einem zweiten Rand (8) an seiner gegenüberliegenden zweiten Stirnfläche, **dadurch gekennzeichnet, daß** der rohrförmige Mantel (1) durch einen bei der Herstellung an wenigstens einem Ende verengten Zylinder gebildet ist.

9. Platzhalter nach Anspruch 7 oder 8, **dadurch gekennzeichnet, daß** der rohrförmige Mantel (1) an seinem einen Ende aufgeweitet und an seinem anderen Ende verengt ist.

10. Platzhalter nach einem der Ansprüche 7 bis 9, **dadurch gekennzeichnet, daß** der Mantel (1) eine Mehrzahl von über die Oberfläche verteilt angeordnete Durchbrechungen bzw. Ausnehmungen (3, 4) aufweist.

11. Platzhalter nach Anspruch 10, **dadurch gekennzeichnet, daß** die Ausnehmungen (3, 4) eine Vielzahl von in Umfangsrichtung einander benachbarter rautenförmiger Ausnehmungen umfassen.

12. Platzhalter nach Anspruch 11, **dadurch gekennzeichnet, daß** die Manteloberfläche die Form eines Rautengitters aufweist.

## Claims

1. Method for manufacturing a tubular spacer which has recesses in its wall and which is used to connect bone parts via its opposite end faces and which has a first rim (7) at its first end, and a second rim (8) at its opposite, second end, said method comprising the following steps:
- forming a cylindrical tube (9) with a length corresponding to the distance between bone parts that are to be connected to one another, and
- expanding the cylindrical tube (9) from at least one end.

2. Method for manufacturing a tubular spacer which has recesses in its wall and which is used to connect bone parts via its opposite end faces and which has a first rim (7) at its first end, and a second rim (8) at its opposite, second end, said method comprising the following steps:
- forming a cylindrical tube (9) with a length corresponding to the distance between bone parts that are to be connected to one another, and
- narrowing the cylindrical tube (9) from at least one end.

3. Method according to either of Claims 1 and 2, **characterized in that** the expansion proceeds from one end and the narrowing proceeds from the other end.

4. Method according to one of Claims 1 to 3, **characterized in that** the cross section of the expanded and/or narrowed part differs from the non-deformed cross section.

5. Method according to one of Claims 1 to 4, **characterized in that** the expansion is brought about by pushing in a mandrel (11).

6. Method according to one of Claims 2 to 5, **characterized in that** the narrowing is brought about by pushing the end into a hollow mould (14).

7. Spacer for connecting bone parts via its opposite end faces, with a tubular jacket (1) which comprises recesses and which has a first rim (7) at its first end face, and a second rim (8) at its opposite, second end face, **characterized in that** the tubular jacket (1) is formed by a cylinder which is expanded at least at one end during manufacture.

8. Spacer for connecting bone parts via its opposite end faces, with a tubular jacket (1) which comprises recesses and which has a first rim (7) at its first end face, and a second rim (8) at its opposite, second end face, **characterized in that** the tubular jacket (1) is formed by a cylinder which is narrowed at least at one end during manufacture.

9. Spacer according to Claim 7 or 8, **characterized in that** the tubular jacket (1) is expanded at one end and is narrowed at its other end.

10. Spacer according to one of Claims 7 to 9, **characterized in that** the jacket (1) has a multiplicity of openings or recesses (3, 4) distributed over its surface.

11. Spacer according to Claim 10, **characterized in that** the recesses (3, 4) comprise a multiplicity of rhomboid-shaped recesses arranged adjacent to each other in the circumferential direction.

12. Spacer according to Claim 11, **characterized in that** the jacket surface has the shape of a rhomboid lattice.

## Revendications

1. Procédé de fabrication d'un écarteur tubulaire, présentant des évidements dans sa paroi, pour relier des parties d'os à ses faces frontales opposées, présentant, sur sa première extrémité, un premier bord (7) et, sur sa deuxième extrémité, opposée, un deuxième bord (8), comprenant les étapes ci-après :
- formation d'un tube (9) à forme cylindrique, avec une longueur correspondant à l'espacement des parties d'os à relier entre elles, et
- élargissement du tube (9) à forme cylindrique depuis au moins une extrémité.

2. Procédé de fabrication d'un écarteur tubulaire, présentant des évidements dans sa paroi, pour assurer la liaison de parties d'os à ses faces frontales opposées, présentant, sur sa première extrémité, un premier bord (7), et, sur sa deuxième extrémité, opposée, un deuxième bord (8), avec les étapes ci-après :
- formation d'un tube (9) à forme cylindrique avec une longueur correspondant à l'espacement des parties d'os à relier entre elles, et
- rétrécissement du tube (9) en forme de cylindre depuis au moins une extrémité.

3. Procédé selon l'une des revendications 1 ou 2, **caractérisé en ce que** l'élargissement se fait depuis une extrémité et le rétrécissement depuis l'autre extrémité.

4. Procédé selon l'une des revendications 1 à 3, **caractérisé en ce que** la section transversale de la partie élargie et/ou rétrécie est différente de la section transversale non déformée.

5. Procédé selon l'une des revendications 1 à 4, **caractérisé en ce que** l'élargissement s'effectue par enfoncement d'un mandrin (11).

6. Procédé selon l'une des revendications 2 à 5, **caractérisé en ce que** le rétrécissement s'effectue par enfoncement de l'extrémité dans une forme creuse (14).

7. Ecarteur pour la liaison de parties d'os à ses faces frontales opposées, avec une enveloppe (1) tubulaire, présentant des évidements, avec un premier bord (7), sur sa première face frontale, et un deuxième bord (8), sur sa deuxième face frontale, opposée, **caractérisé en ce que** l'enveloppe (1) tubulaire est formée par un cylindre, élargi en au moins une extrémité lors de la fabrication.

8. Ecarteur pour la liaison de parties d'os à ses faces frontales opposées, avec une enveloppe (1) tubulaire, présentant des évidements, avec un premier bord (7), sur sa première face frontale, et un deuxième bord (8), sur sa deuxième face frontale, opposée, **caractérisé en ce que** l'enveloppe (1) tubulaire est formée par un cylindre rétréci en au moins une extrémité lors de la fabrication.

9. Ecarteur selon la revendication 7 ou 8, **caractérisé en ce que** l'enveloppe (1) tubulaire est élargie sur une de ses extrémités et rétrécie à son autre extrémité.

10. Ecarteur selon l'une des revendications 7 à 9, **caractérisé en ce que** l'enveloppe (1) présente une pluralité de passages ou d'évidements (3, 4), répartis sur la surface.

11. Ecarteur selon la revendication (10), **caractérisé en ce que** les évidements (3, 4) comprennent une pluralité d'évidements en forme de losanges, voisins les uns des autres en direction périphérique.

12. Ecarteur selon la revendication (11), **caractérisé en ce que** la surface d'enveloppe présente la forme d'une grille de losanges.
